# EUROPÄISCHE PATENTSCHRIFT

(11) **EP 2 089 704 B1**
(45) Veröffentlichungstag und Bekanntmachung des Hinweises auf die Patenterteilung: **09.10.2013**
(21) Anmeldenummer: 07819804.1
(22) Anmeldetag: 14.11.2007
(51) Int. Cl.: G01N 33/00

(54) **VERFAHREN UND VORRICHTUNG ZUR BEHANDLUNG BIOLOGISCHER ZELLEN AUF EINEM SUBSTRAT**
METHOD AND DEVICE FOR TREATING BIOLOGICAL CELLS ON A SUBSTRATE
PROCÉDÉ ET DISPOSITIF POUR LE TRAITEMENT DE CELLULES BIOLOGIQUES SUR UN SUBSTRAT

(30) Priorität: 21.11.2006 DE 102006054789
(43) Veröffentlichungstag der Anmeldung: 19.08.2009
(73) Patentinhaber: Fraunhofer-Gesellschaft zur Förderung der angewandten Forschung e.V., 80686 München (DE)
(72) Erfinder: Zimmermann, Heiko, 66386 St. Ingbert (DE); Ehrhart, Friederike, 66386 St. Ingbert (DE); Fuhr, Günter R., 13187 Berlin (DE)
(74) Vertreter: Hertz, Oliver
(86) Internationale Anmeldenummer: PCT/EP2007/009847
(87) Internationale Veröffentlichungsnummer: WO 2008/061660

(56) Entgegenhaltungen:
- WO-A-2004/046337
- WO-A-2005/063147
- WO-A-2006/011855
- AWERBUCH T E ET AL: "PLATE DIFFUSION ASSAY AS A RAPID METHOD FOR DOSIMETRY OF MUTAGENS" APPLIED AND ENVIRONMENTAL MICROBIOLOGY, Bd. 38, Nr. 6, 1979, Seiten 1127-1131, XP002473459 ISSN: 0099-2240
- DAVIS W W ET AL: "DISC PLATE METHOD OF MICROBIOLOGICAL ANTIBIOTIC ASSAY PART 1 FACTORS INFLUENCING VARIABILITY AND ERROR" APPLIED MICROBIOLOGY, Bd. 22, Nr. 4, 1971, Seiten 659-665, XP002473460 ISSN: 0003-6919
- ITO YOSHIHIRO ET AL: "Micropatterned immobilization of epidermal growth factor to regulate cell function" BIOCONJUGATE CHEMISTRY, Bd. 9, Nr. 2, März 1998 (1998-03), Seiten 277-282, XP002473461 ISSN: 1043-1802

## Beschreibung

Die Erfindung betrifft ein Verfahren zur Behandlung biologischer Zellen auf einem Substrat, insbesondere zur Behandlung biologischer Zellen, die auf einem Substrat eine adhärente Zellkultur bilden. Des Weiteren werden eine Behandlungsvorrichtung zur Behandlung biologischer Zellen auf einem Substrat, insbesondere eine Behandlungsvorrichtung, die zur Durchführung des genannten Verfahrens eingerichtet ist, und eine Zellzusammensetzung, die biologische Zellen enthält, beschrieben.

Aus der Praxis sind zellbiologische Verfahren bekannt, die auf die Manipulation biologischer Zellen in einem suspendierten Zustand oder in einem adhärenten Zustand gerichtet sind. Suspendierte Zellen können vorteilhafterweise berührungslos und unter Ausschluss unerwünschter Zellkontakte behandelt werden. Nachteilig ist jedoch, dass die Umgebungsbedingungen suspendierter Zellen nicht den natürlichen Lebensbedingungen der Zellen entsprechen. Typischerweise befinden sich Zellen eines biologischen Organismus in Verbindung mit einem Zellverband, z. B. einer Zellgruppe oder einem Gewebe in einem adhärenten Zustand. Wenn ein Interesse an einer Anpassung an die natürlichen Umgebungsbedingungen besteht, werden daher zellbiologische Verfahren bevorzugt mit Zellen durchgeführt, die auf einem Substrat angeordnet sind und insbesondere eine adhärente Kultur bilden. In der adhärenten Kultur sind die Zellen als Mono- oder Mehrfachschicht auf dem Substrat angeordnet (sog. Zellrasen), wobei sich die Zellen in der Zellkultur gegenseitig berühren können.

Zellbiologische Verfahren unter Verwendung adhärenter Zellen haben den Nachteil, dass auf Einzelzellen in einer adhärenten Zellkultur nur beschränkt selektiv zugegriffen werden kann. Typischerweise ist es nicht möglich, ausschließlich eine einzelne Zelle in der adhärenten Zellkultur zu behandeln oder zu untersuchen, ohne Nachbarzellen oder die gesamte Zellkultur zu beeinflussen. Beispielsweise ist es bisher nicht möglich, durch Zugabe eines Differenzierungsfaktors in ein Kulturmedium eine Differenzierung ausschließlich einer einzelnen Zelle zu induzieren. Vielmehr werden die Differenzierungsfaktoren eine entsprechende Differenzierung auch bei den Nachbarzellen auslösen. Entsprechend sind auch eine biochemisch induzierte Lyse oder eine Markierung einzelner Zellen in einer adhärenten Zellkultur nicht möglich, ohne auch Nachbarzellen entsprechend zu schädigen oder zu markieren. Durch diese Beschränkung sind zellbiologische Verfahren, insbesondere Hochdurchsatzverfahren und Verfahren, bei denen Zellen einer gemeinsamen Zellkultur verschiedenen Manipulationen oder Untersuchungen ausgesetzt werden sollen, an einzelnen Zellen oder Zellgruppen innerhalb größerer Zellverbände ausgeschlossen.

Behandlungen von adhärenten Zellkulturen sind beispielsweise aus den Veröffentlichungen von Tamara E. Awerbuch and Avishay A. Stark: Applied and Environmental Microbiology, 1979, Vol. 38, Nr. 6, p. 1127-1131; und von W.W. Davis and T.R. Stout in Applied Microbiology, 1971 , Vol. 22, No. 4, p. 659-665 bekannt.

Aus DE 102 03 629 ist bekannt, einzelne Zellen zur Abgrenzung gegenüber der Umgebung in Alginat zu verkapseln. Dieses Verfahren ist jedoch auf die Benutzung einer speziellen Verkapselungseinrichtung beschränkt, mit der nur suspendierte Zellen verkapselt werden können. Das Verfahren ist nicht für die Verkapselung einzelner Zellen in einer adhärenten Zellkultur geeignet. Aus der WO 2005/063147 A1 sind Alginatkörper ferner als Träger für Zellschichten bekannt.

In WO 2004/046337 wird vorgeschlagen, dreidimensionale Zellkulturen auf einem Substrat zu bilden, um Zell-Zell-Wechselwirkungen zu studieren, wobei insbesondere Biopolymer-Schichten gebildet werden. Die herkömmliche Technik ist darauf beschränkt, dass in allen Ebenen (Schichten) der dreidimensionalen Zellkultur Zellen enthalten sind, die miteinander in Wechselwirkung treten können, so dass auf die Zellkultur nicht selektiv zugegriffen werden kann. Somit ist es nicht möglich, ausschließlich einzelne Zelle zu behandeln oder zu untersuchen, ohne Nachbarzellen oder die gesamte Zellkultur zu beeinflussen.

Der Erfindung liegt die Aufgabe zugrunde, ein verbessertes Verfahren zur Behandlung biologischer Zellen bereitzustellen, mit dem die Nachteile der herkömmlichen Techniken überwunden werden.

Diese Aufgabe wird durch ein Verfahren mit den Merkmalen der unabhängigen Ansprüche gelöst. Vorteilhafte Ausführungsformen und Anwendungen der Erfindung ergeben sich aus den abhängigen Ansprüchen.

Verfahrensbezogen wird die genannte Aufgabe insbesondere durch ein Verfahren zur Behandlung biologischer Zellen gelöst, die auf einem Substrat angeordnet sind, wobei eine Abdecksubstanz derart auf den Zellen angeordnet wird, dass mindestens eine Zelle bedeckt ist und mindestens eine Zelle freiliegt. Die Bildung einer lokal selektiven Beschichtung der Zellen mit der Abdecksubstanz wird auch als zweidimensionale Verkapselung bezeichnet.

Die Erfinder haben festgestellt, dass durch die lokal selektive Beschichtung der Zellen mit der Abdecksubstanz eine lokale Maskierung oder Abschirmung der Zellen ermöglicht wird, unter der die mindestens eine abgedeckte Zelle im lebenden Zustand erhalten bleibt. Mit der Abdecksubstanz wird die mindestens eine Zelle gegenüber der Umgebung abgeschirmt. Im Gegensatz zu herkömmlichen, in der Praxis bisher angewendeten Verfahren zur Behandlung von substratgebundenen Zellen werden erfindungsgemäß Zellen oder Zellgruppen (oder komplementär alle Zellen mit Ausnahme einzelner Zellen oder Zellgruppen) verkapselt, so dass für die verkapselten Zellen oder Zellgruppen selektiv die Umgebungsbedingungen verändert werden, während andere Zellen oder Zellgruppen im unveränderten Zustand bleiben. Es wird insbesondere ermöglicht, eine einzelne Zellen verschiedenen Behandlungen auszusetzen, was insbesondere für Hochdurchsatzverfahren und für Referenzzwecke von Vorteil ist. Des Weiteren können mit dem erfindungsgemäßen Verfahren Zellanordnungen auf Substraten lokal verändert, z. B. differenziert werden, wodurch sich Vorteile für die Bereitstellung maßgeschneiderter Zellsysteme oder für das sog. "Tissue Engineering" ergeben.

Zellen, die auf einem Substrat angeordnet sind, umfassen jede Anordnung von biologischen Zellen im lokalisierten Zustand auf einem festen Substrat, die insbesondere einzelne Zellen oder Zellgruppen, die mit gegenseitigen Abständen auf dem Substrat angeordnet sind, dreidimensionale Zellhaufen (z. B. Sphäroide), eine Monoschicht der Zellen auf dem Substrat und/oder eine Mehrfachschicht der Zellen auf dem Substrat umfasst. Allgemein können die Zellen den lokalisierten Zustand auf dem Substrat bilden, indem die Zellen durch eine äußere Einwirkung, insbesondere hochfrequente elektrische Felder, wie zum Beispiel durch Elektrophorese oder Dielektrophorese, ein äußeres Adhäsionsmittel, wie zum Beispiel Poly-L-Lysin, oder durch eine mit dem Substrat gebildete Rezeptor-Ligand-Bindung auf dem Substrat festgehalten werden. Im letzteren Fall bilden die Zellen auf dem Substrat eine adhärente Zellkultur. Die erfindungsgemäße Behandlung wird auf adhärente Zellkulturen angewendet, da mit diesen eine gute Anpassung an natürliche Umgebungsbedingungen erreicht werden kann. Die Zellkultur kann eine heterogene Struktur aufweisen, die mehrere der genannten Arten der Anordnung von Zellen umfasst. Die selektive Beschichtung mit der Abdecksubstanz wird auch bei der Abdeckung dreidimensionaler Zellhaufen als zweidimensionale Verkapselung bezeichnet.

Die Zellen auf dem Substrat sind mit einer Flüssigkeit bedeckt, die insbesondere eine Kultivierungsflüssigkeit oder zur Behandlung der Zellen mit einer biologischen oder chemischen Einwirkung eine Behandlungsflüssigkeit umfasst. Die Flüssigkeitsschicht über der Zellkultur bildet während der Kultivierung oder der Einwirkung einen Überstand über den Zellen. Während der erfindungsgemäß vorgesehenen Bereitstellung der Abdecksubstanz auf der Zellkultur kann die Flüssigkeitsschicht teilweise oder vollständig abgeleitet, z. B. abgesaugt werden, so dass die Oberfläche der Zellen von einer dünnen Flüssigkeitsschicht (Dicke z. B. 3 bis 4 mm) bedeckt ist oder freiliegt. Die Erfinder haben festgestellt, dass sogar nach Ableitung des Flüssigkeitsüberstands die Zellen durch einen restlichen Flüssigkeitsfilm bedeckt bleiben, der die Zellen für die Dauer der Auftragung und Fixierung der Abdecksubstanz im lebensfähigen Zustand erhält. Überraschenderweise kann die Abdecksubstanz ohne die Flüssigkeitsschicht der Kultivierungs- oder Behandlungsflüssigkeit gezielt auf die mindestens eine Zelle, die abgedeckt werden soll, aufgetragen werden, während die Zellen in ihrem lebenden Zustand bleiben. Vorteilhafterweise kann der restliche Flüssigkeitsfilm, der z. B. eine Dicke von rund 10 µm aufweist, von den typischerweise verwendeten Abdecksubstanzen, die z. B. wasserhaltige Gele umfassen, aufgenommen werden. Die Anwendung einer komplexen Verkapselungseinrichtung, wie sie zur Verkapselung suspendierter Zellen bekannt ist, wird vermieden. Das erfindungsgemäße Verfahren zeichnet sich entsprechend vorzugsweise dadurch aus, dass während der Auftragung der Abdecksubstanz auf die mindestens eine Zelle die Zellen ausschließlich von einem durch Zellmembranen gehaltenen Flüssigkeitsfilm bedeckt ist. Nach der Auftragung der Abdecksubstanz wird über den Zellen eine Flüssigkeitsschicht gebildet, die ein Fixiermittel und ggf. eine Kultivierungsflüssigkeit und/oder eine Behandlungsflüssigkeit umfasst.

Die auf dem Substrat mit den Zellen aufgetragene und fixierte Abdecksubstanz bildet ein vorbestimmtes Maskierungsmuster, das vorteilhafterweise in Abhängigkeit von der gewünschten Behandlung gewählt werden kann. Beispielsweise kann gemäß einer ersten Variante der Erfindung vorgesehen sein, dass mindestens eine einzelne Zelle der Gesamtheit der Zellen, insbesondere der Zellkultur mit der Abdecksubstanz bedeckt ist, während die Zellen in der Umgebung der mindestens einen abgedeckten Zelle von der Abdecksubstanz unbedeckt sind (freiliegen). Es kann bspw. ausschließlich eine einzige Zelle abgedeckt sein, während die gesamten übrigen Zellen freiliegen. Vorteilhafterweise kann die abgedeckte Zelle eine Referenzzelle bilden, die bei der optional vorgesehenen Behandlung der Zellen für Vergleichszwecke verwendet werden kann. Alternativ kann eine Vielzahl einzelner Zellen abgedeckt werden, die ein bestimmtes Zellmuster bilden. Vorteilhafterweise kann dann mit der Behandlung der Gesamtheit der Zellen eine heterogene Zellkultur, z. B. mit verschieden differenzierten Zelltypen gebildet werden. Gemäß einer weiteren Variante kann entsprechend vorgesehen sein, dass mindestens eine einzelne Zellgruppe mit der Abdecksubstanz bedeckt wird, während die Umgebung der mindestens einen Zellgruppe freiliegt. Entsprechend werden Vorteile durch die Schaffung einer Referenzzellgruppe oder einer Zellkultur mit heterogenen Zellbereichen erzielt.

Gemäß weiteren Varianten des erfindungsgemäßen Verfahrens kann die Abdecksubstanz so angeordnet werden, dass mindestens eine einzelne Zelle oder mindestens eine einzelne Zellgruppe freiliegt, während Zellen in der Umgebung der mindestens einen, freiliegenden Zelle oder der mindestens einen, freiliegenden Zellgruppe mit der Abdecksubstanz bedeckt sind. Vorteilhafterweise kann bei diesen Varianten die Abdeckung und die optional vorgesehene Behandlung auf vorbestimmte Teilbereiche mit einzelnen Zellen, Gruppen von einzelnen Zellen, einer einzelnen Zellgruppe oder mehreren Zellgruppen innerhalb der Gesamtheit der auf dem Substrat angeordneten Zellen beschränkt werden. Wenn die Behandlung eine Überführung der freiliegenden Zelle oder Zellgruppe in den nicht-adhärenten Zustand (Ablösung der Zelle) umfasst, so können z. B. in einer Zellkultur schonend und ohne eine unerwünschte Beeinflussung der übrigen adhärenten Zellen Lücken gebildet werden, die in weiteren Verfahrensschritten z. B. andere Zelltypen oder synthetische Substanzen aufnehmen können. Mit der Erfindung werden somit neue Möglichkeiten des "Tissue Engineering" geschaffen.

Die Anordnung der Abdecksubstanz auf dem Substrat kann vorteilhafterweise mit jeder Technik zur Beschichtung des Substrats mit der Abdecksubstanz im flüssigen Zustand bereitgestellt werden. Gemäß einer bevorzugten Ausführungsform der Erfindung ist eine lokal selektive Beschichtung der Zellkultur vorgesehen, die bspw. eine lokale Tropfenablage umfasst. Alternativ ist ein Gießen der Abdecksubstanz mindestens auf einen Bereich vorgesehen, der mit einer Maskierungsschablone begrenzt ist. Vorteilhafterweise ist damit eine besonders schonende Auftragung der Abdecksubstanz und optional die weitere Behandlung der Zellkultur unmittelbar nach der Fixierung der Abdecksubstanz möglich.

Die erfindungsgemäß verwendete Maskierungsschablone, die z. B. aus Kunststoff, Glas oder Metall besteht, wird bspw. durch ein Maskierungsgitter gebildet, das während der Auftragung der Abdecksubstanz auf der Zellkultur angeordnet ist. Das Maskierungsgitter deckt bestimmte Zellen während der Auftragung der Abdecksubstanz ab. Alternativ wird die Maskierungsschablone bspw. durch mindestens eine Maskierungshülse gebildet, die während der Auftragung der Abdecksubstanz auf die Zellkultur, vorzugsweise unmittelbar auf das Substrat aufgesetzt ist. Die Maskierungshülse, die z. B. aus dem Polymer PDMS oder Metall besteht und z. B. einen runden (Maskierungsring) oder eckigen Querschnitt aufweist, eignet sich insbesondere für das Aufgießen der Abdecksubstanz. Nach der Fixierung der Abdecksubstanz kann die Maskierungsschablone vorteilhafterweise einfach abgehoben werden, um die nicht von der Abdecksubstanz bedeckten Zellen freizulegen.

Alternativ oder zusätzlich kann die Maskierungsschablone eine Maskierungsschicht umfassen, die auf dem Substrat mit den Zellen gebildet ist. Die Maskierungsschicht besteht z. B. aus einem Polymer oder einer zähen Flüssigkeit. Gemäß einer vorteilhaften Variante der Erfindung wird als Polymer ein Material verwendet, das als Abdecksubstanz zur erfindungsgemäßen Verkapselung von Zellen geeignet ist. Beispielsweise können innerhalb der Gesamtheit der Zellen auf dem Substrat verschiedene Zellen oder Zellgruppen mit Abdecksubstanzen mit verschiedenen Verkapselungseigenschaften abgedeckt werden. Die verschiedenen Abdecksubstanzen können sich beispielsweise in Bezug auf die Durchlässigkeit für Wirkstoffe in einer optional vorgesehenen chemischen oder biologischen Behandlung, insbesondere einem Porendurchmesser unterscheiden. Erfindungsgemäß kann entsprechend die in einem ersten Verfahrenszyklus vorgesehene Verkapselung von Zellen zur Maskierung einer in einem weiteren Verfahrenszyklus vorgesehenen weiteren Verkapselung von Zellen verwendet werden. Die optional zur Bildung der Maskierungsschicht verwendete zähe Flüssigkeit zeichnet sich dadurch aus, dass sie nach einer lokal selektiven Deposition auf dem Substrat mit den Zellen, z. B. unter Verwendung eines Plotters oder eines Tropfendispensers, nicht auseinanderfließt. Hierzu wird beispielsweise ein zähes Öl, z. B. Silikonöl verwendet.

Gemäß einer alternativen Ausführungsform der Erfindung kann eine gleichmäßige Beschichtung der gesamten Zellen vorgesehen sein, wobei in diesem Fall nach der Fixierung der Abdecksubstanz eine selektive Abtragung der Abdecksubstanz entsprechend dem gewünschten Maskierungsmuster vorgesehen ist. Die Abtragung kann z. B. mit einem mechanisch wirkenden Werkzeug, einer Lichtbestrahlung oder einer chemischen Ablösung erreicht werden. Die gleichmäßige Beschichtung der Zellen mit einer anschließenden selektiven Abtragung hat den Vorteil, dass die Zufuhr der Abdecksubstanz, z. B. durch Aufschleudern oder unmaskiertes Gießen vereinfacht und durch die selektive Abtragung die Bildung komplexer Maskierungsmuster ermöglicht wird.

Mit "Abdecksubstanz" wird jede biokompatible Substanz bezeichnet, die im flüssigen Zustand auf die Zellkultur aufgetragen und physikalisch oder chemisch in einen fixierten Zustand überführt werden kann. Im fixierten Zustand bildet die Abdecksubstanz eine formhaltige, feste oder gelförmige Abdeckschicht.

Gemäß einer bevorzugten Ausführungsform der Erfindung wird als Abdecksubstanz eine Alginatlösung verwendet, die durch Zuführung einer zweiwertigen Ionenlösung fixierbar (gelierbar) ist. Die Verwendung des ionotropen Gels Alginat hat besondere Vorteile, da die Kompatibilität von Alginat mit einer Vielzahl biologischer Zellen bekannt ist. Des Weiteren hat Alginat die vorteilhafte Eigenschaft, Nährstoffe zur Versorgung der bedeckten Zelle durchzulassen, während das Eindringen größerer Moleküle, wie z. B. von Proteinen des Immunsystems verhindert wird. Gemäß alternativen Ausführungsformen der Erfindung werden als Abdecksubstanzen andere ionotrope Gele, thermotrope Gele, z. B. Gelatine oder Agar, pH-Wertgeschaltete Gele (siehe z. B. TongTrinh in "Sensors and Actuators" Bd. 117, 2006, S. 17-26), Enzym-geschaltete Gele (z. B. Nap-FFGEY) oder Kollagen-basierte Gele, wie z. B. Matri-Gel oder Cy-Gel (Handelsbezeichnungen) verwendet.

Die Verwendung von Alginat zur schichtförmigen Abdeckung von substratgebundenen Zellen, insbesondere von adhärent wachsenden biologischen Zellen stellt einen unabhängigen Gegenstand der Erfindung dar.

Es kann insbesondere Barium-gebundenes Alginat und/oder Kalzium-gebundenes Alginat verwendet werden. Barium-gebundenes Alginat kann für eine dauerhafte Abdeckung von Zellen vorteilhaft sein. Kalzium-gebundenes Alginat hingegen ist besonders für lösbare Abdeckschichten geeignet. Die Ablösung kann z. B. mit Citrat-Lösung erfolgen, um in einer Zusammensetzung aus Zellen und Abdecksubstanz beispielsweise Kanalstrukturen oder Schwammstrukturen zu bilden.

Die Fixierung der Abdecksubstanz ist nach oder während der Auftragung der Abdecksubstanz auf den Zellen vorgesehen. Die Vielzahl verfügbarer biokompatibler Abdecksubstanzen ergibt vorteilhafterweise eine Variabilität bei der Auswahl eines geeigneten und an die konkrete Anwendung angepassten Fixierungstyps. Vorzugsweise ist zur Fixierung eine Gelierung, Ausfällung oder Aushärtung. Wenn diese Fixierungstypen durch eine Bestrahlung, eine Temperierung (Abkühlung oder Erwärmung) der Abdecksubstanz oder eine Zufuhr eines Fixierungsmittels in flüssiger oder fester Form umfasst, ergeben sich Vorteile für eine schnelle und gezielte Verfahrensführung.

Die Abdecksubstanz bildet vorzugsweise eine Schichtdicke im Bereich von 2 µ bis 10 mm, bevorzugt 10 µm bis 500 µm, besonders bevorzugt im Bereich von 20 µm bis 200 µm. Die Erfinder haben festgestellt, dass für Schichtdicken außerhalb dieser Bereiche eine ausreichende Abschirmung der Zellen gegen eine äußere physikalische, chemische oder biologische Einwirkung oder eine ausreichende Versorgung der Zellen mit Nährstoffen nicht erreicht werden kann. Allerdings ist es gemäß einer alternativen Ausführungsform der Erfindung möglich, die Abdeckschicht mit einer größeren Schichtdicke, z. B. im mm- bis zum cm-Bereich zu bilden, wenn die Zellkultur auf einem Substrat angeordnet ist, das für Nährstoffe durchlässig ist. Die Zellkultur kann bspw. auf einer Membran zur Nährstoffversorgung angeordnet sein.

Erfindungsgemäß kann insbesondere durch mehrere Verfahrenszyklen, umfassend jeweils eine Verkapselung von Zellen und optional eine weitere Behandlung der Zellen und/oder der aufgetragenen Abdecksubstanz eine Schichtzusammensetzung gebildet werden, die in mehreren Schichten Zellen und Abdecksubstanz enthält. Die Schichtzusammensetzung kann eine Dicke bis in den mm- bis cm-Bereich aufweisen und bevorzugte Anwendungen beim "Tissue Engineering" haben.

Die erfindungsgemäße Anordnung der fixierten Abdecksubstanz auf dem Substrat mit den Zellen kann als solche bereits eine Behandlung der Zellen, z. B. durch eine Trennung von verkapselten Zellen von einem Kultivierungsmedium oder von benachbarten Zellen umfassen. Gemäß einer besonders bevorzugten Ausführungsform der Erfindung ist eine weitere Behandlung der Zellen nach deren lokal selektiver Verkapselung vorgesehen.

Die weitere Behandlung der Zellen umfasst jede Prozedur, durch die ein Zustand der Zellen erfasst (gemessen, detektiert), erhalten oder verändert wird. Entsprechend umfasst die Behandlung insbesondere physikalische, chemische oder biologische Modifizierungen der Zellen oder Untersuchungen der Zellen durch physikalische, chemische oder biologische Untersuchungsverfahren.

Gemäß einer bevorzugten Ausführungsform der Erfindung umfasst die Behandlung der Zellen eine chemische oder biologische Einwirkung, die durch die Zufuhr einer Behandlungslösung induziert wird. Vorteilhafterweise können somit an sich bekannte Perfusionstechniken zur Flüssigkeitsbehandlung biologischer Zellen angewendet werden. Die Behandlungslösung umfasst bspw. eine Lösung einer Wirksubstanz oder eine Suspension mit biologischen wirksamen Partikeln, insbesondere eine Suspension biologischer Zellen, z. B. dendritischer Zellen, oder eine Suspension von Zellbestandteilen, z. B. mit Membranteilen von Tumorzellen.

Zur Bildung einer erfindungsgemäßen Schichtzusammensetzung aus Zellen und Verkapselungssubstanzen kann die Behandlung die Kultivierung von Zellen auf den lokal selektiv verkapselten Zellen umfassen. Entsprechend kann das Substrat mit den lokal selektiv verkapselten Zellen als solches das Substrat für einen weiteren Verfahrenszyklus zur Zellkultivierung und/oder für eine weitere erfindungsgemäße Behandlung bilden.

Vorrichtungsbezogen wird eine Behandlungsvorrichtung zur Behandlung biologischer Zellen beschrieben, die eine Kultureinrichtung mit einem Substrat zur Aufnahme von Zellen, insbesondere einer adhärenten Zellkultur, eine Beschichtungseinrichtung zur Zufuhr der Abdecksubstanz auf die Zellkultur und eine Fixiereinrichtung zur Fixierung der Abdecksubstanz umfasst. Vorteilhafterweise wird mit der Behandlungsvorrichtung ein vielseitig anwendbarer, einfach strukturierter Aufbau bereitgestellt, der für die selektive Behandlung von einzelnen Zellen oder Zellgruppen innerhalb von Zellkulturen geeignet ist. Als Beschichtungseinrichtung können vorteilhafterweise an sich in der Labortechnik verfügbare Geräte, wie z. B. eine Gießeinrichtung, eine Schleudereinrichtung, eine Tropfpipette oder eine Kolbenspritze verwendet werden. Die Fixiereinrichtung kann in Abhängigkeit von der Art der verwendeten Abdecksubstanz gewählt sein und z. B. eine Temperiereinrichtung, eine Spüleinrichtung zur Zufuhr einer Gelierlösung oder eine Beleuchtungseinrichtung umfassen. Die Temperiereinrichtung kann als zusätzliche Komponente an der Kultureinrichtung oder an der Beschichtungseinrichtung vorgesehen sein.

Gemäß einer Variante ist die Behandlungsvorrichtung mit einer Kameraeinrichtung zur Bildaufnahme an der Kultureinrichtung ausgestattet. Die Kameraeinrichtung ist dazu eingerichtet, Bilder der Zellkultur auf dem Substrat aufzunehmen. Die Bilder können von einer Hauptsteuereinrichtung verarbeitet und zur Bildung von Steuersignalen für die Beschichtungseinrichtung zur lokal selektiven Zufuhr der Abdecksubstanz auf die Zellkultur verwendet werden. Vorteilhafterweise kann damit die Behandlungsvorrichtung für einen automatischen Betrieb ausgelegt sein.

Das Substrat der Behandlungsvorrichtung umfasst vorzugsweise ein von den üblichen Verfahren der Zellbiologie bekanntes, festes Substrat mit einer ebenen oder gekrümmten Oberfläche, wie z. B. ein Glassubstrat mit einer Poly-L-Lysin-Oberfläche. Alternativ kann ein für Nährstoffe durchlässiges Substrat, wie z. B. eine Membran, z. B. ein Polyethylensulfon- oder Polykarbonatfilter verwendet werden. Zur Bildung von Schichtzusammensetzungen aus Zellen und Verkapselungssubstanzen können auf dem Substrat bereits Zellen vorhanden sein. Es können insbesondere lokal selektiv verkapselte Zellen ein- oder mehrschichtig vorhanden sein.

Vorrichtungsbezogen wird ferner eine Zellzusammensetzung beschrieben, die biologische Zellen und mindestens eine Abdecksubstanz umfasst, mit der mindestens eine Zelle gegenüber der Umgebung abgedeckt ist. Vorzugsweise bildet die Zellzusammensetzung eine Schichtzusammensetzung mit mehreren Schichten aus selektiv verkapselten Zellen auf einem Substrat.

Weitere Einzelheiten und Vorteile der Erfindung werden im Folgenden unter Bezug auf die beigefügten Zeichnungen beschrieben. Es zeigen:
- Figuren 1 bis 3:: verschiedene Ausführungsformen erfindungsgemäßer Verfahren zur Behandlung biologischer Zellen;
- Figur 4:: verschiedene Varianten der Auftragung einer Substanz auf eine Zellkultur;
- Figuren 5 bis 7:: verschiedene Ausführungsformen der erfindungsgemäßen vorgesehenen Behandlung der Zellkultur;
- Figur 8:: eine schematische Schnittansicht einer Zellzusammensetzung; und
- Figur 9:: eine Behandlungsvorrichtung (keine Ausführungsform der Erfindung).

Bevorzugte Ausführungsformen der Erfindung werden im Folgenden unter beispielhaftem Bezug auf die Verkapselung adhärenter Zellen beschrieben. In diesem Fall bilden die Zellen mit dem Substrat Rezeptor-Ligand-Bindungen. Die Umsetzung der Erfindung ist nicht auf die bevorzugte Behandlung adhärenter Zellen beschränkt, sondern alternativ entsprechend mit Zellen möglich, die mit anderen Mitteln, wie z. B. unter der Wirkung von Adhäsionsmitteln oder elektrischen Feldern auf dem Substrat lokalisiert angeordnet sind.

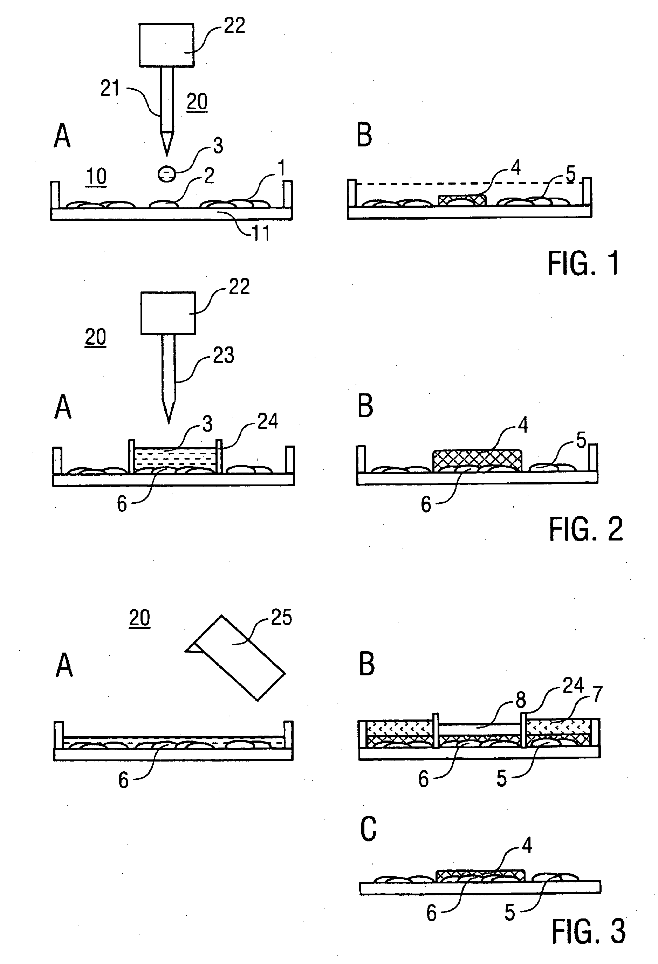

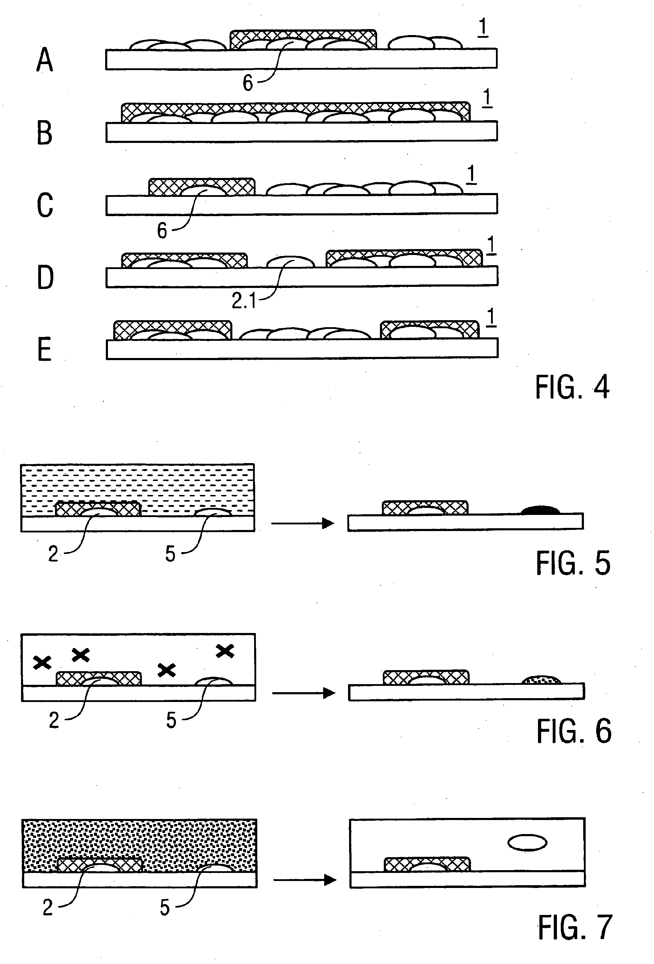

Das erfindungsgemäße Verfahren zur Behandlung adhärenter Zellen umfasst die örtlich beschränkte, zweidimensionale Verkapselung einzelner Zellen oder Zellgruppen einer Zellkultur und optional die anschließende Behandlung der Zellkultur mit einer physikalischen oder chemischen Einwirkung. Im Folgenden werden in den Figuren 1 bis 4 zunächst bevorzugte Ausführungsbeispiele der Verkapselung der Zellen oder Zellgruppen beschrieben. Beispiele für die Behandlung der Zellkultur werden dann unter Bezug auf die Figuren 5 bis 7 beschrieben. Es wird beispielhaft auf die Behandlung einer Zellkultur auf einem ebenen Glassubstrat Bezug genommen. Die Umsetzung der Erfindung ist jedoch nicht auf diese Ausführungsform beschränkt, sondern entsprechend auch mit anderen Substratformen und/oder Materialien möglich.

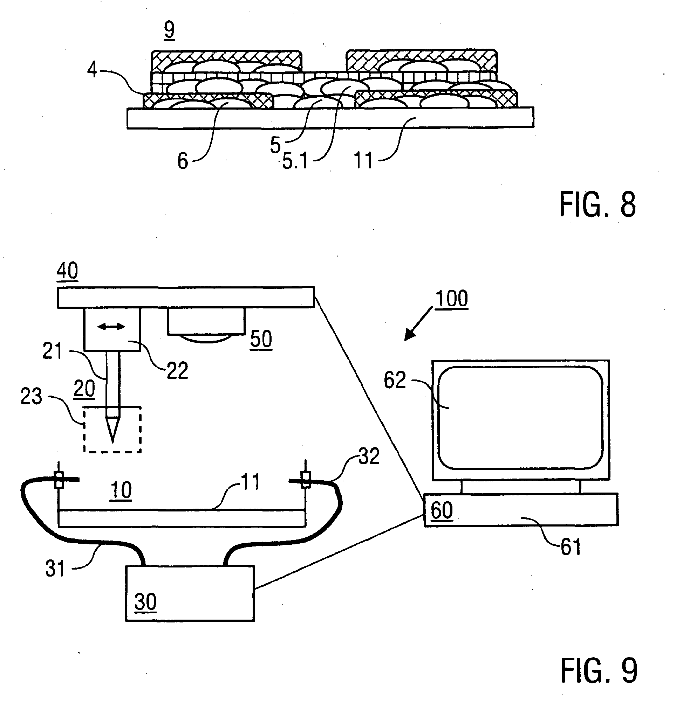

Figur 1A zeigt in schematischer Schnittansicht eine Kultureinrichtung 10 mit einer Zellkultur 1 und eine Beschichtungseinrichtung 20. Die Kultureinrichtung 10 umfasst ein Kulturgefäß, dessen Boden ein Substrat 11 zur Aufnahme der Zellkultur 1 bildet. Das Kulturgefäß kann in an sich bekannter Weise mit weiteren Komponenten, wie z. B. Flüssigkeitszufuhr- und -abfuhreinrichtungen ausgestattet sein, die in Figur 1A nicht gezeigt sind. Die Beschichtungseinrichtung 20 umfasst eine Tropfpipette 21, die mit einer Fluidikeinrichtung 22 verbunden ist. Die Fluidikeinrichtung 22 enthält ein Flüssigkeitsreservoir, das zur Aufnahme einer flüssigen Abdecksubstanz eingerichtet ist, und eine Steuereinrichtung, die zur Zufuhr der Abdecksubstanz in die Tropfpipette 21 und zur Betätigung der Tropfpipette 21 eingerichtet ist. Des Weiteren kann die Fluidikeinrichtung 22 eine Antriebseinrichtung enthalten, die zur Bewegung der Tropfpipette 21 relativ zu einer ortsfesten Halteeinrichtung (siehe Figur 8, Bezugszeichen 40) eingerichtet ist.

Die Bildung der Zellkultur 1 auf dem Substrat 11 erfolgt entsprechend zellbiologischen Verfahren, die an sich aus der Laborpraxis bekannt sind. Beispielsweise wird das Substrat 11 aus Glas mit einer hydrophilen Beschichtung und einer Poly-L-Lysin-Beschichtung versehen. Alternativ kann Glas mit einer Alginat-Beschichtung vorgesehen sein. Auf der Poly-L-Lysin- oder der Alginat-Oberfläche werden adhärente Zellen, wie z. B. Stammzellen, neuronale Zellen, dendritische Zellen oder Tumorzellen mit einer Kultivierungsflüssigkeit kultiviert. Die kultivierte Zellkultur 1 enthält bspw. eine einzelne Zelle 2, die einen Abstand von Zellen der umgebenden Zellkultur aufweist. Der Abstand kann als Ergebnis des natürlichen Zellwachstums oder eines Präparationsschrittes gebildet sein, bei dem Nachbarzellen der einzelnen Zelle 2 mechanisch abgetragen werden.

Die zweidimensionale Verkapselung der einzelnen Zelle 2 umfasst die folgenden Schritte. Zunächst wird die Position der Zelle 2 innerhalb der Zellkultur 1 erfasst und die Beschichtungseinrichtung 20 so ausgerichtet, dass die Tropfpipette 21 auf die Zelle 2 gerichtet ist. Die Positionserfassung erfolgt bspw. durch eine visuell-mikroskopische Beobachtung der Zellkultur 1 durch eine Bedienperson oder automatisch unter Verwendung einer Kameraeinrichtung (siehe Figur 8, Bezugszeichen 50). Zur Beschichtung der Zelle 2 mit der Abdecksubstanz wird zunächst die Kultivierungsflüssigkeit in der Kultureinrichtung 10 entfernt. Die Zellkultur 1 wird mit Pufferlösung, z. B. PBS (Phosphat-gepufferte Salzlösung), gewaschen. Es sind bspw. drei Waschvorgänge vorgesehen. Abschließend wird die Flüssigkeit aus der Kultureinrichtung 10 entfernt, so dass die Zellkultur 1 im wesentlichen frei liegt und nur von einem restlichen Flüssigkeitsfilm bedeckt ist. In diesem Zustand, der in Figur 1A gezeigt ist, wird die Zelle 2 mit der Abdecksubstanz 3 betropft. Es wird mindestens ein Tropfen der Abdecksubstanz 3 (rd. 5 pl bis 5 µl, insbesondere 100 pl bis 25 µl) auf die Zelle 2 aufgetragen. Die Menge der Flüssigkeit, d. h. die Anzahl der Tropfen kann in Abhängigkeit von den Anforderungen der konkreten Anwendung der zweidimensionalen Verkapselung und insbesondere unter Berücksichtigung der Viskosität der Abdecksubstanz 3 gewählt werden. Die Abdecksubstanz 3 umfasst z. B. eine flüssige Alginatlösung.

Nach der gerichteten Betropfung der Zelle 2 folgt die Fixierung der Abdecksubstanz, die beim beschriebenen Beispiel eine Polymerisierung des Alginats umfasst. Hierzu wird eine Polymerisierungslösung aufgetragen, die zwei-wertige Kationen, z. B. Ba²⁺ oder Ca²⁺ enthält. Die Auftragung der Polymerisierungslösung kann auf der gesamten Zellkultur 1 oder beschränkt im Bereich der Zelle 2 mit der Abdecksubstanz 3 erfolgen. Zur Polymerisierung folgt eine Inkubation bei Raumtemperatur für rd. 20 Minuten. Im Ergebnis ist auf der Zelle 2 eine Abdeckschicht 4 gebildet, die räumlich auf die Zelle 2 beschränkt ist. Zellen 5 in der Umgebung der Zelle 2 sind von der Abdeckschicht 4 nicht bedeckt. Diese Zellen 5 bilden eine freie Zellkulturoberfläche. Dieser Zustand ist in Figur 1B gezeigt.

Nach der Bildung der Abdeckschicht 4 wird die Zellkultur 1 mit PBS gewaschen. Es sind bspw. drei Waschvorgänge vorgesehen. Anschließend werden die Zellen mit einer Kultivierungsflüssigkeit überschichtet (in Figur 1B gestrichelt gezeigt) oder unmittelbar einer Behandlung unterzogen, z. B. wie sie unten unter Bezug auf die Figuren 5 bis 7 erläutert ist.

Alternativ zur genannten Polymerisierung kann bei Verwendung einer thermisch vernetzenden Abdecksubstanz (z. B. Gelatine, Agar) zur Fixierung der Abdecksubstanz eine Heizeinrichtung, eine Kühleinrichtung oder eine Beleuchtungseinrichtung, z. B. zur UV-Bestrahlung, vorgesehen sein. Wenn als Beschichtungseinrichtung ein geheizter Tropfengeber verwendet wird, der die Abdecksubstanz im erwärmten, flüssigen Zustand abgibt, erfolgt die Fixierung durch die Abkühlung der Abdecksubstanz auf dem Substrat. Die Kühlung wird durch die Umgebung der Kultureinrichtung bereitgestellt. Alternativ kann eine aktive Kühlung an der Kultureinrichtung, z. B. mit einem Peltier-Element vorgesehen sein. Die Abkühlung erfolgt vorzugsweise bis zu einer Temperatur, bei der die Abdecksubstanz geliert, jedoch Wasserbestandteile in der Abdecksubstanz nicht kristallisieren. Die Abkühlung erfolgt entsprechend vorzugsweise bis zu einer Temperatur oberhalb des Gefrierpunkts von Wasser. Alternativ kann als Beschichtungseinrichtung ein Tropfengeber verwendet werden, der bei Raumtemperatur betrieben wird und die Abdecksubstanz im flüssigen Zustand abgibt, wobei die Fixierung durch eine Erwärmung der Abdecksubstanz auf dem Substrat erfolgt. In diesem Fall werden thermotrope Gele, wie z. B. Cy-Gel oder Matri-Gel (Handelsbezeichnungen) verwendet, die durch eine Temperaturerhöhung geliert werden.

In Figur 2 ist eine zweidimensionale Verkapselung einer Zellgruppe 6 gezeigt, wobei eine Beschichtungseinrichtung 20 verwendet wird, die eine Kolbenspritze 23 und als Maskierungsschablone einen Maskierungsring 24 umfasst. Der Maskierungsring 24 wird auf das Substrat 11 aufgesetzt. Vorzugsweise berührt der untere Umfangsrand des Maskierungsringes 24 unmittelbar die Oberfläche des Substrats 11. Das Aufsetzen des Maskierungsringes 24 auf die Oberfläche des Substrats 11 kann mit der in DE 103 07 487 beschriebenen Technik erfolgen, bei der Zellen verletzungsfrei verdrängt werden.

Mit dem aufgesetzten Maskierungsring 24 erfolgt die Auftragung der Abdecksubstanz und deren Fixierung mit den oben unter Bezug auf Figur 1 beschriebenen Schritten. Die Kolbenspritze 23 wird mit der Fluidikeinrichtung 22 so betätigt, dass über der Zellgruppe 6 eine geschlossene Schicht der Abdecksubstanz 3 gebildet wird (Figur 2A). Die Zufuhr der Polymerisierungslösung kann unter Verwendung des Maskierungsringes 24 auf die Abdecksubstanz 3 beschränkt werden. Nach der Polymerisierung wird der Maskierungsring 24 entfernt, so dass gemäß Figur 2B die Zellgruppe 6 mit der Deckschicht 4 bedeckt ist, während die übrigen Zellen 5 freiliegen. Anschließend folgt die Zuführung des Kultivierungsmediums oder unmittelbar die Behandlung der partiell abgedeckten Zellkultur 1.

Figur 3 illustriert eine weitere Variante der ortsselektiven, zweidimensionalen Verkapselung einer Zellgruppe 6, wobei zunächst durch Betropfung der gesamten Zellkultur 1 mit einer Gießeinrichtung 25 der Beschichtungseinrichtung 20 eine gleichmäßige Flüssigkeitsschicht der Abdecksubstanz 3 gebildet wird (Figur 3A). Anschließend wird mit einem Maskierungsring 24 die zu verkapselnde Zellgruppe 6 von den übrigen Zellen 5 abgegrenzt. Die Abdecksubstanz außerhalb der Zellgruppe 6 wird mit einer Waschlösung 7 (z. B. isotone Kochsalzlösung) entfernt und über der Zellgruppe 6 mit einer Polymerisierungslösung 8 polymerisiert (Figur 3B). Alternativ kann eine Polymerisierung der gesamten Schicht der Abdecksubstanz 3 und anschließend eine ortsselektive Abtragung der polymerisierten Schicht vorgesehen sein. In diesem Fall wird an Stelle der Waschlösung 7 ein Lösungsmittel, z. B. Zitrat zu Ablösung der polymerisierten Abdecksubstanz über den Zellen 5 verwendet. Im Ergebnis bleibt die Abdeckschicht 4 über der Zellgruppe 6 stehen, während die übrigen Zellen 5 freiliegen (Figur 3C).

Figur 4 zeigt verschiedene Varianten der erfindungsgemäßen zweidimensionalen Verkapselung adhärenter Zellkulturen mit einer Verkapselung einer einzelnen Zellgruppe 6 (Figur 4A, siehe auch Figur 2, 3), der gesamten Zellkultur 1 (Figur 4B), einer einzelnen Zelle 2 (Figur 4C, siehe auch Figur 1), oder der gesamten Zellkultur 1 mit Ausnahme einer einzelnen Zelle 2.1 (Figur 4D) oder einer einzelnen Zellgruppe 6.1 (Figur 4E). Diese Varianten können im Rahmen der Erfindung modifiziert werden, indem bspw. Muster einzelner Zellen 2, 2.1 oder einzelner Zellgruppen 6, 6.1 exklusiv frei bleiben. Die Zellgruppen 6, 6.1 können als homogener Teil der Zellkultur 1, d. h. als Zellschicht gebildet sein. Des Weiteren kann die Zellkultur 1 eine heterogene Struktur aufweisen, die innerhalb einer Zellschicht Sphäroide enthält. Dreidimensionale Zellhaufen können entsprechend mit den oben beschriebenen Techniken selektiv verkapselt werden.

Nach der Verkapselung der Zellkultur 1 ist erfindungsgemäß eine Behandlung durch eine chemische oder physikalische Einwirkung vorgesehen. Eine chemische Einwirkung wird vorzugsweise durch eine Behandlungsflüssigkeit induziert, die vorbestimmte Wirkkomponenten enthält. Die physikalische Einwirkung umfasst z. B. eine Bestrahlung der Zellkultur derart, dass nur die freiliegenden Zellen der Bestrahlung ausgesetzt sind.

Die Figuren 5 bis 7 zeigen Beispiele chemischer Einwirkungen, die aus Gründen der Klarheit mit einzelnen Zellen illustriert sind.

Die in Figur 5 illustrierte Behandlung der Zellkultur umfasst eine gezielte Lyse durch ein Komplementsystem. Die gesamte Zellkultur mit abgedeckten Zellen 2 und freiliegenden Zellen 5 (z. B. Fibroblasten oder andere Zellen, die nicht mit dem Komplementsystem, insbesondere dem Immunsystem des Spenders des betreffenden Blutplasmas kompatibel sind) wird mit einer Behandlungsflüssigkeit überschichtet, die humanes Blutplasma umfasst. Die im Blutplasma enthaltenen Komponenten des Komplementsystems töten innerhalb kurzer Zeit (rd. 1 h) die freiliegenden Zellen 5 ab (Figur 5, rechte Seite). Alternativ kann die Behandlungslösung andere chemische Stoffe zum gezielten Abtöten der adhärenten Zellen, wie z. B. isolierte T-Killerzellen enthalten. Die Erfinder haben festgestellt, dass die Wirkung des Blutplasmas oder alternativ verwendeten Substanzen durch die Abdeckschicht 4 der Abdecksubstanz unterbunden wird.

Die Behandlung der Zellkultur 1 kann alternativ eine gezielte Differenzierung der freiliegenden Zellen 5 umfassen, wie beispielhaft in Figur 6 illustriert ist. Zur gezielten Behandlung von undifferenzierten Stammzellen 2, 5 enthält die Behandlungslösung einen differenzierenden Faktor, z. B. Retinsäure, der die Abdeckschicht 4 nicht durchdringen kann. Durch die Behandlung mit dem differenzierenden Faktor wird in der freiliegenden Zelle 5 die Differenzierung ausgelöst, während die abgedeckte Zelle 2 im undifferenzierten Zustand bleibt. Die selektive Differenzierung innerhalb einer Zellkultur wurde bspw. mit Neuroblastomazellen (N2a-Zellen) demonstriert. Bei einer Behandlung mit Retinsäure wurde in den unbeschichteten Zellen 5 eine Differenzierung zu Neuronenzellen induziert, während die abgedeckten Zellen 2 undifferenziert blieben.

Gemäß Figur 7 umfasst die Behandlung der Zellkultur eine gezielte Ablösung einzelner Zellen 5 oder Zellgruppen. In diesem Fall enthält die Behandlungsflüssigkeit Proteinenzyme, die zum Ablösen von adhärenten Zellen verwendet werden, wie z. B. eine Mischung aus Trypsin und EDTA. Es wurde im Experiment festgestellt, dass mit Alginat beschichtete Zellen 2 z. B. 5 min in Trypsin/EDTA-Lösung inkubiert werden, ohne dass sie abgelöst werden, während die freibleibenden Zellen 5 abgelöst werden.

Nach der Behandlung kann mindestens einer der folgenden Schritte, ggf. in Kombination mit einer Entfernung der Abdeckschicht vorgesehen sein. Erstens kann die Zellkultur mit einer Kultivierungsflüssigkeit weiter kultiviert werden. Zweitens kann auf der Zellkultur eine weitere Zellkultur, ggf. mit einem anderen Zelltyp gebildet werden. Drittens kann der Verfahrenszyklus der erfindungsgemäßen Abdeckung und Behandlung der Zellkultur mindestens einfach wiederholt werden, wobei ggf. andere Zellen oder Zellgruppen oder Maskierungsmuster abgedeckt werden oder frei bleiben oder andere Behandlungen ausgeführt werden. Schließlich können Zellen oder Zellgruppen, ggf. im Verbund mit einer Abdecksubstanz einer Kryokonservierung unterzogen werden.

Die Bildung einer dreidimensionalen Schichtzusammensetzung 7 umfassend abgedeckte Zellen 6, die eine Schicht der Abdecksubstanz 4 tragen und freiliegende Zellen 5, auf denen Zellen 5.1 einer weiteren Zellschicht angeordnet sind, ist beispielhaft in Figur 8 gezeigt. Die Zellzusammensetzung 7 kann erfindungsgemäß mit dem Substrat 11 verbunden oder von diesem abgelöst sein.

Figur 9 zeigt beispielhaft eine Behandlungsvorrichtung 100 mit der Kultureinrichtung 10, der Beschichtungseinrichtung 20, einer Fixierungseinrichtung 30, einer Halteeinrichtung 40, einer Kameraeinrichtung 50 und einer Hauptsteuereinrichtung 60. Die Kultureinrichtung 10 und die Beschichtungseinrichtung 20 sind bspw. so aufgebaut, wie dies oben unter Bezug auf die Figuren 1 bis 3 beschrieben wurde.

Die Fixierungseinrichtung 30 umfasst z. B. ein Flüssigkeitsreservoir, das zur Aufnahme einer Fixierlösung eingerichtet und über eine Zufuhrleitung 31 und einer Abfuhrleitung 32 mit der Kultureinrichtung 10 verbunden ist. Mit der Fixiereinrichtung 30 wird bspw. die Polymerisierungslösung zum Polymerisieren von Alginat in die Kultureinrichtung 10 geleitet. Alternativ ist als Fixierungseinrichtung eine Heizeinrichtung, eine Kühleinrichtung oder eine Bestrahlungseinrichtung vorgesehen.

Die Halteeinrichtung 40 umfasst einen Träger für die Beschichtungseinrichtung 20 und die Kameraeinrichtung 50. Die Beschichtungseinrichtung 20 und ggf. auch die Kameraeinrichtung 50 ist an der Halteeinrichtung 40 verstellbar angeordnet. Die Kameraeinrichtung 50 umfasst eine Vergrößerungsoptik, z. B. ein Mikroskop und eine CCD-Kamera zur Aufnahme von Bildern die Zellkultur auf dem Substrat 11 der Kultureinrichtung 10.

Die Hauptsteuereinrichtung 60 enthält eine Datenverarbeitungseinheit 61 und eine Anzeigeeinrichtung 62. Für eine automatisierte Behandlung von Zellkulturen ist die Hauptsteuereinrichtung 60 zur Steuerung der Beschichtungseinrichtung 20, der Fixierungseinrichtung 30 und ggf. der Kameraeinrichtung 50 eingerichtet.

Die in der vorstehenden Beschreibung, den Ansprüchen und den Zeichnungen offenbarten Merkmale der Erfindung können sowohl einzeln als auch in Kombination für die Verwirklichung der Erfindung in ihren verschiedenen Ausgestaltungen von Bedeutung sein.

## Patentansprüche

1. Verfahren zur Behandlung biologischer Zellen (1) auf einem Substrat (11), mit den Schritten:
- Bildung einer adhärenten Zellkultur der Zellen (1) auf dem Substrat (11), und
- Anordnung einer biokompatiblen Abdecksubstanz (3) auf dem Substrat (11) derart, dass mindestens eine Zelle (2, 6) bedeckt ist und mindestens eine Zelle (5, 2.1, 6.1) freiliegt, zur Bildung einer lokal selektiven Beschichtung der Zellen (1),
**dadurch gekennzeichnet, dass**
- die Abdecksubstanz im flüssigen Zustand auf die Zellkultur aufgetragen wird, und
- eine Fixierung der Abdecksubstanz (3) vorgesehen ist, wobei die Abdecksubstanz (3) physikalisch oder chemisch in einen fixierten Zustand überführt wird, in dem die Abdecksubstanz eine formhaltige, feste oder gelförmige Abdeckschicht bildet.

2. Verfahren gemäß Anspruch 1, bei dem die Anordnung der Abdecksubstanz (3) eine Bildung eines Maskierungsmusters der Abdecksubstanz (3) derart umfasst, dass
- mindestens eine einzelne Zelle (2) bedeckt ist, wobei die Umgebung der mindestens einen einzelnen Zelle (2) freiliegt,
- mindestens eine Zellgruppe (6) bedeckt ist, wobei die Umgebung der mindestens einen Zellgruppe (6) freiliegt,
- mindestens eine einzelne Zelle (2.1) freiliegt, wobei die Umgebung der mindestens einen Zelle (2.1) mit der Abdecksubstanz bedeckt ist, oder
- mindestens eine Zellgruppe (6.1) freiliegt, wobei eine Umgebung der mindestens einen Zellgruppe mit der Abdecksubstanz bedeckt ist.

3. Verfahren gemäß Anspruch 1 oder 2, bei dem die Anordnung der Abdecksubstanz (3)
- eine lokal selektive Beschichtung des Substrats (11), oder
- eine gleichmäßige Beschichtung des Substrats (11) und eine anschließende selektive Abtragung der Abdecksubstanz (3) vom Substrat (11) umfasst.

4. Verfahren gemäß Anspruch 3, bei dem
- die lokal selektive Beschichtung eine lokal selektive Tropfenablage oder ein Gießen der Abdecksubstanz (3) in mindestens einen mit einer Maskierungsschablone abgegrenzten Bereich, oder
- die gleichmäßige Beschichtung ein Aufschleudern oder ein Gießen der Abdecksubstanz (3) umfasst.

5. Verfahren gemäß mindestens einem der vorhergehenden Ansprüche, bei dem die Abdecksubstanz ein ionotropes Gel, ein thermotropes Gel, ein pH-Wert-geschaltetes Gel, ein Enzymgeschaltetes Gel oder ein Kollagen-basiertes Gel umfasst.

6. Verfahren gemäß Anspruch 5, bei dem die Abdecksubstanz Alginat umfasst.

7. Verfahren gemäß mindestens einem der vorhergehenden Ansprüche, bei dem die Fixierung der Abdecksubstanz eine Gelierung und/oder eine Ausfällung umfasst.

8. Verfahren gemäß Anspruch 7, bei dem zur Fixierung eine Bestrahlung, eine Abkühlung oder eine Erwärmung der Abdecksubstanz oder eine Flüssigkeits- oder Feststoffzufuhr zu der Abdecksubstanz vorgesehen ist.

9. Verfahren gemäß mindestens einem der vorhergehenden Ansprüche, bei dem
- die Abdecksubstanz über der mindestens einen abgedeckten Zelle (2, 6) eine Schichtdicke im Bereich von 2 µm bis 10 mm aufweist,
- bei der Anordnung der Abdecksubstanz die mindestens eine Zelle (2, 6) von einem Flüssigkeitsfilm bedeckt ist,
- eine Behandlung der Zellen (1) mit mindestens einer physikalischen, chemischen oder biologischen Einwirkung vorgesehen ist, und/oder
- die Behandlung der Zellen (1) eine Zufuhr einer Behandlungsflüssigkeit auf die Zellen (1) umfasst.

10. Verfahren gemäß Anspruch 9, bei dem
- die Behandlungsflüssigkeit mindestens einen Differenzierungsfaktor, mindestens ein Enzym, weitere biologische Zellen und/oder Zellbestandteile enthält, und/oder
- die Behandlung eine Kultivierung der mit der Behandlungsflüssigkeit zugeführten biologischen Zellen umfasst.

11. Verfahren gemäß mindestens einem der vorhergehenden Ansprüche, bei dem nach der Behandlung die Schritte:
- Anordnung und Fixierung einer weiteren Abdecksubstanz auf den Zellen vorgesehen sind.

12. Verfahren gemäß Anspruch 11, mit dem Schritt:
- weitere Behandlung der Zellen mit mindestens einer physikalischen, chemischen oder biologischen Einwirkung.

## Claims

1. A method for the treatment of biological cells (1) on a substrate (11), with the steps of:
- formation of an adherent cell culture of the cells (1) on the substrate (11), and
- arrangement of a biocompatible covering substance (3) on the substrate (11) so that at least one cell (2, 6) is covered and at least one cell (5, 2.1, 6.1) lies exposed, for the formation of a locally selective coating of the cells (1),
**characterized in that**
- the covering substance is applied in a liquid state to the cell culture, and
- fixation of the covering substance (3) is provided, wherein the covering substance (3) is transformed physically or chemically into a fixed state in which the covering substance forms a dimensionally stable, solid or gel-like covering layer.

2. The method according to claim 1, in which the arrangement of the covering substance (3) comprises a formation of a masking pattern of the covering substance (3) in such a way that
- at least one individual cell (2) is covered, wherein the environment of the at least one individual cell (2) lies exposed,
- at least one cell group (6) is covered, wherein the environment of the at least one cell group (6) lies exposed,
- at least one individual cell (2.1) lies exposed, wherein the environment of the at least one individual cell (2.1) is covered with the covering substance, or
- at least one cell group (6.1) lies exposed, wherein the environment of the at least one cell group is covered with the covering substance.

3. The method according to claim 1 or 2, in which the arrangement of the covering substance (3) comprises
- a locally selective coating of the substrate (11), or
- a uniform coating of the substrate (11) and a subsequent selective removal of the covering substance (3) from the substrate (11).

4. The method according to claim 3, in which
- the locally selective coating comprises a locally selective drop deposition or pouring of the covering substance (3) in at least one area delimited by a masking screen, or
- the uniform coating comprises spin-coating or pouring of the covering substance (3).

5. The method according to at least one of the preceding claims, in which the covering substance comprises an ionotropic gel, a thermotropic gel, a pH-switched gel, an enzyme-switched gel or a collagen-based gel.

6. The method according to claim 5, in which the covering substance comprises alginate.

7. The method according to at least one of the preceding claims, in which the fixation of the covering substance comprises a gelation and/or a precipitation.

8. The method according to claim 7, in which radiation, cooling or heating of the covering substance or supply of a liquid or solid substance to the covering substance is provided for fixing.

9. The method according to at least one of the preceding claims, in which
- the covering substance has a layer thickness in the range of 2 µm up to 10 mm above the at least one covered cell (2, 6),
- for the arrangement of the covering substance, the at least one cell (2, 6) is covered by a liquid film,
- a treatment of the cells (1) with at least one physical, chemical or biological action is provided, and/or
- the treatment of the cells (1) comprises a supply of a treatment liquid to the cells (1).

10. The method according to claim 9, in which
- the treatment liquid contains at least one differentiation factor, at least one enzyme, further biological cells and/or cell constituents, and/or
- the treatment comprises a cultivation of the biological cells supplied with the treatment liquid.

11. The method according to at least one of the preceding claims, in which, after the treatment, the steps of:
- arrangement and fixing of a further covering substance on the cells are provided.

12. The method according to claim 11, with the step of:
- further treatment of the cells with at least one physical, chemical or biological action.

## Revendications

1. Procédé de traitement de cellules biologiques (1) sur un substrat (11) comprenant les étapes de :
- formation d'une culture cellulaire adhérente des cellules (1) sur le substrat (11), et
- disposition d'une substance de recouvrement biocompatible (3) sur le substrat (11), de telle sorte qu'au moins une cellule (2, 6) soit recouverte et qu'au moins une cellule (5, 2.1, 6.1) soit découverte, pour la formation d'un revêtement localement sélectif des cellules (1),
**caractérisé en ce que**
- la substance de recouvrement est appliquée à l'état liquide sur la culture cellulaire, et
- une fixation de la substance de recouvrement (3) est prévue, la substance de recouvrement (3) étant convertie physiquement ou chimiquement en un état fixé dans lequel la substance de recouvrement forme une couche de recouvrement conservant sa forme, solide ou formant un gel.

2. Procédé selon la revendication 1, dans lequel la disposition de la substance de recouvrement (3) comprend une formation d'un modèle de masquage de la substance de recouvrement (3) de telle sorte que
- au moins une cellule unique (2) soit recouverte, l'environnement de l'au moins une cellule unique (2) étant découvert,
- au moins un groupe de cellules (6) soit recouvert, l'environnement de l'au moins un groupe de cellules (6) étant découvert,
- au moins une cellule unique (2.1) soit découverte, l'environnement de l'au moins une cellule (2.1) étant recouvert par la substance de recouvrement, ou
- au moins un groupe de cellules (6.1) soit découvert, un environnement de l'au moins un groupe de cellules étant recouvert avec la substance de recouvrement.

3. Procédé selon la revendication 1 ou 2, dans lequel la disposition de la substance de recouvrement (3)
- comprend un revêtement localement sélectif du substrat (11) ou
- un revêtement uniforme du substrat (11) et un retrait sélectif consécutif de la substance de recouvrement (3) du substrat (11).

4. Procédé selon la revendication 3, dans lequel
- le revêtement localement sélectif comprend un dépôt de gouttes localement sélectif ou un coulage de la substance de recouvrement (3) dans au moins une zone délimitée avec un gabarit de masquage, ou
- le revêtement uniforme comprend un dépôt par centrifugation ou un coulage de la substance de recouvrement (3).

5. Procédé selon au moins l'une des revendications précédentes, dans lequel la substance de recouvrement est un gel ionotrope, un gel thermotrope, un gel commandé par le pH, un gel commandé par une enzyme ou un gel à base de collagène.

6. Procédé selon la revendication 5, dans lequel la substance de recouvrement comprend l'alginate.

7. Procédé selon au moins l'une des revendications précédentes, dans lequel la fixation de la substance de recouvrement comprend une gélification et/ou une précipitation.

8. Procédé selon la revendication 7, dans lequel, pour la fixation, un rayonnement, un refroidissement ou un réchauffement de la substance de recouvrement ou un apport de liquide ou de solide à la substance de recouvrement est prévu.

9. Procédé selon au moins l'une des revendications précédentes, dans lequel
- la substance de recouvrement présente sur l'au moins une cellule recouverte (2, 6) une épaisseur de couche dans une plage de 2 µm à 10 mm,
- dans de la disposition de la substance de recouvrement, l'au moins une cellule (2, 6) est recouverte par un film de liquide,
- un traitement des cellules (1) avec au moins une action physique, chimique ou biologique est prévu, et/ou
- le traitement des cellules (1) comprend un apport d'un liquide de traitement sur les cellules (1).

10. Procédé selon la revendication 9, dans lequel
- le liquide de traitement contient au moins un facteur de différenciation, au moins une enzyme, d'autres cellules biologiques et/ou composants cellulaires, et/ou
- le traitement comprend une culture des cellules biologiques acheminées avec le liquide de traitement.

11. Procédé selon au moins l'une des revendications précédentes, dans lequel après le traitement, les étapes de :
- disposition et fixation d'une autre substance de recouvrement sur les cellules, sont prévues.

12. Procédé selon la revendication 11, comportant l'étape de :
- autre traitement des cellules avec au moins une action physique, chimique ou biologique.
